(19) Europäisches Patentamt
European Patent Office
Office européen des brevets

(11) EP 4 265 211 A1

(12) EUROPÄISCHE PATENTANMELDUNG

(43) Veröffentlichungstag:
25.10.2023 Patentblatt 2023/43

(51) Internationale Patentklassifikation (IPC):
$A61B\ 18/16$ (2006.01)

(21) Anmeldenummer: 22169248.6

(22) Anmeldetag: 21.04.2022

(52) Gemeinsame Patentklassifikation (CPC):
A61B 18/16; A61B 18/1233; A61B 18/14;
A61B 2018/00577; A61B 2018/00589;
A61B 2018/00601; A61B 2018/00648;
A61B 2018/00684; A61B 2018/0072;
A61B 2018/00767; A61B 2018/00869;
A61B 2018/00875; A61B 2018/1253;
A61B 2018/128; A61B 2018/167

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
KH MA MD TN

(71) Anmelder: Erbe Elektromedizin GmbH
72072 Tübingen (DE)

(72) Erfinder: MAIER, Philipp
72074 Tuebingen (DE)

(74) Vertreter: Rüger Abel Patentanwälte PartGmbB
Webergasse 3
73728 Esslingen a. N. (DE)

(54) **ELEKTROCHIRURGIESYSTEM UND VERFAHREN ZUR ERMITTLUNG EINES ELEKTRODENTYPS EINER NEUTRALELEKTRODE**

(57) Die Erfindung betrifft ein Elektrochirurgiesystem (15) sowie ein Verfahren (50). Das Elektrochirurgiesystem (15) und das Verfahren (50) sind dazu eingerichtet, einen Elektrodentypen (T1, T2, T3) einer verwendeten Neutralelektrode (23) zu ermitteln. Hierzu wird an die Neutralelektrode (23) ein alternierendes Messsignal (M) bei wenigstens zwei unterschiedlichen Messfrequenzen (f1, f2) angelegt. Bei jeder Messfrequenz (f1, f2) wird ein Impedanzbetragswert ($ZN_{abs}$) und/oder ein Phasenwert ($\varphi$) für eine Neutralelektrodenimpedanz (ZN) der Neutralelektrode (23) ermittelt. Es wird zumindest ein Impedanzbetragswert ($ZN_{abs}$) und zumindest ein Phasenwert ($\varphi$) ermittelt. Basierend auf dem wenigstens einen Impedanzbetragswert ($ZN_{abs}$) und dem wenigstens einen Phasenwert ($\varphi$) wird der Elektrodentyp (T1, T2, T3) der angeschlossenen Neutralelektrode 23 ermittelt, insbesondere durch einen Vergleich mit bekannten Vergleichswerten.

Fig. 1

EP 4 265 211 A1

**Beschreibung**

Die Erfindung betrifft ein Elektrochirurgiesystem sowie ein Verfahren zur Ermittlung eines Elektrodentyps einer Neutralelektrode. Bei dem Elektrochirurgiesystem handelt es sich insbesondere um ein System mit einem monopolaren Instrument und einer Neutralelektrode, die an ein Versorgungsgerät des Elektrochirurgiesystems anschließbar oder angeschlossen sind. Die Neutralelektrode ist dazu eingerichtet, elektrisch leitend an einem Patienten angebracht zu werden, beispielsweise durch eine Klebeverbindung. Das Verfahren kann unter Verwendung eines erfindungsgemäßen Elektrochirurgiesystems durchgeführt werden. Das Elektrochirurgiesystem kann insbesondere zur Durchführung des erfindungsgemäßen Verfahrens eingerichtet sein.

Bei elektrochirurgischen Instrumenten muss während der Behandlung eines Patienten der Stromkreis vom Versorgungsgerät zur Arbeitselektrode des Instruments und von dort zurück zum Versorgungsgerät geschlossen sein. Zu diesem Zweck ist bei einem monopolaren Instrument eine weitere Elektrode, die als Neutralelektrode bezeichnet wird, am Patienten angebracht. Der Strom kann somit vom Versorgungsgerät zur Arbeitselektrode, von der Arbeitselektrode in den Patienten, durch das Gewebe des Patienten zur Neutralelektrode und von dort zurück zum Versorgungsgerät fließen.

Bei Elektrochirurgiesystemen mit monopolaren Instrumenten können unterschiedliche Elektrodenarten oder Elektrodentypen von Neutralelektroden verwendet werden. Die unterschiedlichen Elektrodentypen der Neutralelektroden können unterschiedlich große Flächeninhalte des wenigstens einen elektrisch leitfähigen Elektrodenbereichs (Kontaktfläche) aufweisen, der am Patienten anliegt. Der wenigstens eine elektrisch leitfähige Elektrodenbereich der Neutralelektrode kann bei unterschiedlichen Elektrodentypen auch verschiedene Geometrien bzw. Dimensionen in einer Längsrichtung und/oder einer Querrichtung aufweisen.

Die Stromdichte an der Neutralelektrode darf nicht zu groß werden, um endogene Verbrennungen während des Betriebs des Elektrochirurgiesystems zu vermeiden.

EP 2 537 479 A1 beschreibt die Steuerung eines medizinischen Geräts in Abhängigkeit von der Neutralelektrodenimpedanz. Die Impedanz wird mit einem vorgegebenen Schwellenwert verglichen und der Betrieb des Elektrochirurgiesystems bzw. des Behandlungsinstruments wird nur dann freigegeben, wenn die Impedanz der Neutralelektrode in einem vorgegebenen Bereich liegt, z.B. maximal 140 Ohm beträgt. Dadurch kann geprüft werden, ob die Neutralelektrode korrekt am Patienten angebracht ist und eine ausreichend gute elektrische Verbindung zum Patienten besteht.

Die Messung von Impedanzen von biologischem Gewebe ist auf dem Gebiet der Impedanzspektroskopie bekannt. Bei der Impedanzspektroskopie sollen Gewebetypen unterschieden werden, die mittels des elektrochirurgischen Instruments des Elektrochirurgiesystems behandelt werden. Beispielsweise beschreibt EP 1 289 415 A1 ein solches Verfahren. Mittels des Instruments wird ein Prüfsignal bei unterschiedlichen Frequenzen an das behandelte Gewebe angelegt und anhand der gemessenen Impedanz wird das behandelte Gewebe charakterisiert. Auf diese Weise ist die Unterscheidung von verschiedenen Gewebetypen möglich. Ein ähnliches Verfahren ist auch in EP 0 813 387 A1 beschrieben.

Bei dem aus WO 03/060462 A2 bekannten Verfahren wird ein elektrischer Puls in das Gewebe eines Patienten eingeleitet und die Reflexion des eingeleiteten Impulses wird erfasst. Anhand der Reflexion soll in Echtzeit gesundes von krankem Gewebe unterschieden werden.

Ein Elektrochirurgiesystem mit einer Messeinheit ist in EP 3 496 638 A1 beschrieben. Dort wird eine Impedanzmessung bei verschiedenen Frequenzen des behandelten Gewebes mittels einer Messeinrichtung durchgeführt. Mittels der Messeinrichtung kann ein Messsignal an das Gewebe angelegt werden. Eine Umschalteinrichtung dient dazu, zwischen einer Spannung zur Behandlung des Gewebes und dem Messsignal umzuschalten. Mittels des Messsignals wird die Impedanz des Gewebes bei unterschiedlichen Frequenzen ermittelt.

Ein Verfahren zur Ermittlung eines lokalen Gewebetyps eines Körpergewebes und ein entsprechendes Elektrochirurgiesystems sind in DE 10 2019 209 333 A1 beschrieben. Zur Gewebebestimmung wird ein Messsignal (Wechselspannung oder Wechselstrom) in das Gewebe eingekoppelt. Die Frequenz des Messsignals kann variieren. Basierend darauf kann ein Impedanzspektrum erfasst und daraus wiederum der Gewebetyp abgeleitet werden.

Es kann als Aufgabe der vorliegenden Erfindung angesehen werden, ein Elektrochirurgiesystem und ein Verfahren zu schaffen, das den Einsatz einer Neutralelektrode verbessert und insbesondere die Sicherheit beim Einsatz der Neutralelektrode erhöht.

Diese Aufgabe wird durch ein Elektrochirurgiesystem mit den Merkmalen des Patentanspruches 1 sowie ein Verfahren mit den Merkmalen des Patentanspruches 15 gelöst.

Das erfindungsgemäße Elektrochirurgiesystem weist ein Versorgungsgerät mit einem Neutralanschluss und eine an dem Neutralanschluss angeschlossene Neutralelektrode auf. Die Neutralelektrode ist dazu eingerichtet, elektrisch leitend mit einem Patienten verbunden zu werden. Zu diesem Zweck kann die Neutralelektrode auf die Haut des Patienten aufgeklebt oder anderweitig befestigt werden.

Das Versorgungsgerät kann dazu eingerichtet sein, ein alternierendes Messsignal bei wenigstens zwei unterschiedlichen Messfrequenzen an den Neutralanschluss anzulegen. Das Messsignal kann ein Wechselspannungsmesssignal oder ein Wechselstrommesssignal sein. Beispielsweise kann das Messsignal sinusförmig bzw. cosinusförmig, dreieckförmig, sägezahnförmig oder rechteckförmig sein.

Wenn das Messsignal eine eingeprägte Wechselmessspannung ist, wird dadurch ein Wechselmessstrom durch die Neutralelektrode bzw. einen Elektrodenstromkreis bewirkt. Wenn das Messsignal ein eingeprägter Wechselmessstrom ist, wird dadurch eine Wechselmessspannung an der Neutralelektrode bzw. am Elektrodenstromkreis bewirkt. Der Elektrodenstromkreis umfasst einen geschlossenen Stromkreis vom Versorgungsgerät zur Neutralelektrode durch das Gewebe des Patienten und zurück zum Versorgungsgerät. Vorzugsweise ist das Instrument nicht Bestandteil des Elektrodenstromkreises und der Elektrodenstromkreis wird durch wenigstens zwei separate elektrisch leitfähige Elektrodenbereiche der Neutralelektrode geschlossen, die jeweils sowohl mit dem Versorgungsgerät als auch mit dem Patienten elektrisch leitend verbunden sind. Somit kann der Elektrodenstromkreis von einem Neutralanschluss des Versorgungsgeräts zu einem ersten Elektrodenbereich, über das Gewebe des Patienten zu einem zweiten Elektrodenbereich und zurück zum Neutralanschluss des Versorgungsgeräts führen.

Das alternierende Messsignal kann insbesondere ein sich periodisch änderndes Messsignal sein. Das Messsignal kann seine Polarität und damit die Stromflussrichtung alternierend ändern. Alternativ dazu könnte das Messsignal auch stets positiv oder stets negativ sein.

Das Messsignal bewirkt also einen Wechselmessstrom und eine Wechselmessspannung an der Neutralelektrode bzw. am Neutralanschluss des Versorgungsgeräts, woraus wiederum ein Impedanzbetragswert einer Neutralelektrodenimpedanz der Neutralelektrode (Scheinwiderstand) und ein Phasenwert der Neutralelektrodenimpedanz ermittelt werden kann. Der Impedanzbetragswert wird für zumindest eine der Messfrequenzen ermittelt und der Phasenwert wird für zumindest eine der Messfrequenzen ermittelt. Bei jeder verwendeten Messfrequenz wird der Impedanzbetragswert und/oder der Phasenwert ermittelt.

Basierend auf dem wenigstens einen Impedanzbetragswert und dem wenigstens einen Phasenwert kann der Elektrodentyp der angeschlossenen Neutralelektrode ermittelt bzw. erkannt werden. Hierzu können Vergleichswerte für den Phasenwert und den Impedanzbetragswert bei den verwendeten Messfrequenzen abgelegt sein (beispielsweise in Form eine Tabelle oder in Form von einer oder mehreren Vergleichskurven), so dass durch einen Vergleich der Elektrodentyp der Neutralelektrode erkannt werden kann.

Durch die Erkennung des Elektrodentyps kann das Versorgungsgerät vorzugsweise automatisch oder alternativ manuell in einen Betriebsmodus geschaltet werden, der an den Elektrodentyp der Neutralelektrode angepasst ist. Sind mehrere Betriebsmodi möglich, können diese dem Bediener zur Auswahl vorgegeben werden und/oder es kann ein Standardmodus daraus automatisch ausgewählt werden. Mittels des wenigstens einen, an den Elektrodentyp angepassten Betriebsmodus kann beispielsweise die elektrische Leistung oder der Arbeitsstrom eingestellt und/oder begrenzt werden. Die Stromdichte am Gewebe des Patienten unterhalb der Neutralelektrode ist vom Elektrodentyp abhängig, insbesondere von dem Gesamtflächeninhalt des wenigstens einen elektrisch leitfähigen Elektrodenbereichs. Die Stromdichte kann somit angepasst an die Größe und/oder Geometrie des Elektrodentyps begrenzt werden. Beispielsweise kann abhängig vom ermittelten Elektrodentyp ein Grenzwert für die elektrische Leistung und/oder den durch die Neutralelektrode fließenden Strom verwendet werden.

Die Ermittlung des Elektrodentyps gestattet zusätzlich oder alternativ zur Ermittlung oder Begrenzung der Stromdichte das Ermitteln von wenigstens einem weiteren Parameter, der beim Betrieb des Versorgungsgeräts verwendet werden kann. Der wenigstens eine weitere Parameter kann beispielsweise aus der folgenden Gruppe ausgewählt werden: eine Temperatur des Gewebes des Patienten in dem Bereich, in dem die Neutralelektrode angebracht ist; ein Flächeninhalt der tatsächlichen Anlagefläche zwischen der Neutralelektrode und dem Patienten; das Vorhandensein eines Kontaktgels zwischen der Neutralelektrode und dem Patienten.

Es ist vorteilhaft, wenn das Versorgungsgerät dazu eingerichtet ist, zumindest einen Phasenwert bei einer anderen Messfrequenz zu ermitteln als den wenigstens einen Impedanzbetragswert.

Es ist vorteilhaft, wenn das Versorgungsgerät dazu eingerichtet ist, für eine erste Messfrequenz einen Impedanzbetragswert und für eine zweite Messfrequenz einen Phasenwert der Neutralelektrodenimpedanz zu ermitteln. Die zweite Messfrequenz ist kleiner als die erste Messfrequenz. Die erste Messfrequenz wird vorzugsweise aus einem Bereich von 5,0 kHz bis 1,0 MHz ausgewählt. Die zweite Messfrequenz kann aus einem Bereich von 100 Hz bis 5,0 kHz ausgewählt werden. Optional kann bei der zweiten Messfrequenz zusätzlich auch ein Impedanzbetragswert ermittelt werden und/oder bei der ersten Messfrequenz kann zusätzlich ein Phasenwert ermittelt werden. Es ist alternativ aber auch möglich, bei der ersten Messfrequenz ausschließlich einen Impedanzbetragswert und bei der zweiten Messfrequenz ausschließlich einen Phasenwert zu ermitteln.

Die Amplitude des Messsignals kann begrenzt werden, insbesondere bei einer Messung zur Bestimmung des Phasenwertes und/oder bei der Messung mit einer der Messfrequenzen, beispielsweise der zweiten Messfrequenz. Die Amplitude der Wechselmessspannung ist vorzugsweise kleiner als 5 V. Die Amplitude eines Wechselstrommesssignals ist insbesondere kleiner als 1,0 mA.

Das Elektrochirurgiesystem kann eine Mehrzahl von Neutralelektroden unterschiedlichen Elektrodentyps aufweisen, von denen eine Neutralelektrode eines Elektrodentyps ausgewählt und an das Versorgungsgerät angeschlossen werden kann. Jeder Elektrodentyp kann mehrere, beispielsweise zwei oder drei, separate Elektrodenbereiche aufweisen. Die Elektrodenbereiche sind in der Neutralelektrode nicht kurzgeschlossen. Sie können unterschiedliche elektrische Potentiale aufweisen. Innerhalb der Neutralelektrode sind die Elektrodenbereiche in Bezug auf die auftretenden elektrischen Ströme und Spannungen vorzugsweise elektrisch voneinander isoliert. Eine elektrische Verbindung zwischen zwei Elektrodenbereichen besteht insbesondere ausschließlich mittelbar über das Gewebe des Patienten, wenn die Neutralelektrode am Patienten angebracht ist.

Die Elektrodentypen können sich durch einen oder mehrere Typparameter voneinander unterscheiden, z.B.:

- die Anzahl der vorhandenen elektrisch leitfähigen Elektrodenbereiche;
- der Gesamtflächeninhalt aller vorhandenen elektrisch leitfähigen Elektrodenbereiche;
- die Kontur oder Geometrie des wenigstens einen elektrisch leitfähigen Elektrodenbereichs;
- das für den wenigstens einen Elektrodenbereich verwendete elektrisch leitfähige Material;
- die Relativposition von zwei oder mehr vorhandenen Elektrodenbereichen relativ zueinander.

Beispielsweise weist wenigstens ein Elektrodentyp der Neutralelektrode genau zwei elektrisch leitfähige Elektrodenbereiche auf. Ein anderer Elektrodentyp der Neutralelektrode weist genau drei elektrisch leitfähige Elektrodenbereiche auf.

Vorzugsweise sind wenigstens zwei der vorhandenen Elektrodenbereiche über jeweils einen Leiter mit dem Neutralanschluss des Versorgungsgeräts verbunden bzw. verbindbar. Beispielsweise können ein erster Elektrodenbereich über einen ersten Leiter und ein zweiter Elektrodenbereich über einen zweiten Leiter mit dem Neutralanschluss des Versorgungsgeräts elektrisch verbunden sein. Die beiden Leiter sind für die auftretenden Ströme und Spannungen elektrisch voneinander isoliert.

Zumindest bei einem der Elektrodentypen oder bei allen Elektrodentypen können ein erster Elektrodenbereich und ein zweiter Elektrodenbereich symmetrisch zu einer Bezugsebene angeordnet sein.

Bei einem Ausführungsbeispiel können der erste Elektrodenbereich und der zweite Elektrodenbereich gleichgroße Flächeninhalte und/oder die gleiche Geometrie aufweisen.

Der erste Elektrodenbereich und der zweite Elektrodenbereich können mit Abstand zueinander angeordnet sein und beispielsweise über einen elektrisch nicht leitfähigen Steg der Neutralelektrode voneinander getrennt sein.

Zumindest bei einem Elektrodentyp hat die Neutralelektrode einen elektrisch leitfähigen dritten Elektrodenbereich. Der dritte Elektrodenbereich ist für die auftretenden Ströme und Spannungen innerhalb der Neutralelektrode elektrisch von allen anderen Elektrodenbereichen, beispielsweise dem ersten Elektrodenbereich und dem zweiten Elektrodenbereich, elektrisch isoliert.

Der dritte Elektrodenbereich kann bei zumindest einem Elektrodentyp den ersten Elektrodenbereich und den zweiten Elektrodenbereich umschließen. In seiner Erstreckungsrichtung ist der dritte Elektrodenbereich vorzugsweise in mehreren Abschnitten gekrümmt und/oder abgewinkelt. Es ist bevorzugt, wenn der dritte Elektrodenbereich in seiner Erstreckungsrichtung zwischen seinen beiden Enden unterbrechungslos ausgeführt ist. Der dritte Elektrodenbereich kann beispielsweise einen an einer einzigen Öffnungsstelle unterbrochenen Verlauf haben, wobei sich die beiden Enden an der Öffnungsstelle gegenüberliegen. Beispielsweise kann der dritte Elektrodenbereich U-förmig oder C-förmig sein. An der Öffnungsstelle des dritten Elektrodenbereichs kann ein Anschlussbereich für die vom dritten Elektrodenbereich umschlossenen Elektrodenbereiche vorhanden sein, beispielsweise ein Anschlussbereich für den ersten Leiter am ersten Elektrodenbereich und für den zweiten Leiter am zweiten Elektrodenbereich.

Der Flächeninhalt des dritten Elektrodenbereichs kann kleiner sein als der Flächeninhalt jedes anderen vorhandenen Elektrodenbereichs, insbesondere des ersten Elektrodenbereichs und des zweiten Elektrodenbereichs. Die Länge des dritten Elektrodenbereichs in seiner Erstreckungsrichtung zwischen den beiden Enden kann vorzugsweise um den Faktor 10 oder 20 größer sein als der Betrag seiner maximalen Breite rechtwinklig zu dieser Erstreckungsrichtung (Länge).

Verschiedene Elektrodentypen können sich zumindest auch dadurch voneinander unterscheiden, wie eine Außenkante des ersten Elektrodenbereichs und des zweiten Elektrodenbereichs ausgeführt ist, die auf den einander abgewandten Außenseiten der Elektrodenbereiche vorhanden ist. Bei einem Elektrodentypen können diese Außenkanten bogenförmig gekrümmt sein und/oder keinen geradlinigen Abschnitt aufweisen und/oder keinen parallel zu der Bezugsebene verlaufenden Abschnitt aufweisen, wobei die Bezugsebene mittig zwischen den beiden Elektrodenbereichen verläuft und eine Symmetrieebene bilden kann. Bei einem anderen Elektrodentypen können die Außenkanten einen geradlinigen Abschnitt aufweisen und/oder einen parallel zur Bezugsebene verlaufenden Abschnitt aufweisen.

Es ist vorteilhaft, wenn das Versorgungsgerät einen Versorgungsanschluss und ein Instrument aufweist, das an den Versorgungsanschluss angeschlossen oder anschließbar ist. Das an den Versorgungsanschluss angeschlossene Instrument wird über das Versorgungsgerät mit einem Arbeitssignal und somit einer elektrischen Arbeitsleitung versorgt, wobei als Arbeitssignal eine Arbeitsspannung oder ein Arbeitsstrom eingeprägt werden kann. Das Instrument hat eine Arbeitselektrode, der das Arbeitssignal zugeführt wird. Während der Verwendung des Elektrochirurgiesystems kann mit Hilfe des Instruments Gewebe des Patienten koaguliert, geschnitten, abladiert oder fusioniert werden. Abhängig von der Funktion kann das Arbeitssignal (Arbeitsspannung und/oder der Arbeitsstrom) variieren, beispielsweise im Hinblick auf die Arbeitsfrequenz, die Amplitude, die Wellenform oder eine beliebige Kombination davon.

Das Versorgungsgerät kann dazu eingerichtet sein, das Messsignal nur dann an den Neutralanschluss anzulegen, wenn der Arbeitselektrode keine Arbeitsspannung und/oder kein Arbeitsstrom zugeführt wird. Das Messsignal liegt an der Neutralelektrode somit nur dann an, wenn über die Neutralelektrode kein Rückfluss des Stromes erfolgt, der über die Arbeitselektrode des Instruments in das Gewebe des Patienten eingeleitet wird. Dadurch wird die Ermittlung des Impedanzbetragswertes und des Phasenwertes nicht durch einen Arbeitsstrom beeinträchtigt, der einen Stromfluss durch die Neutralelektrode bewirkt.

Zusätzlich oder alternativ ist es auch möglich, zumindest phasenweise gleichzeitig Gewebe mittels der Arbeitselektrode zu behandeln (an der Arbeitselektrode liegt eine Spannung an und/oder es fließt ein Strom durch die Arbeitselektrode) und das Messsignal an den Neutralanschluss anzulegen. In den Phasen oder Zeiträumen, in denen sich eine Behandlungsperiode und das Anlegen des Messsignals überlappen, kann die Messfrequenz des Messsignals verschieden sein von der Frequenz des Arbeitssignals (Arbeitsspannung und/oder Arbeitsstrom). Dadurch ist sichergestellt, dass sich der durch das Wechselspannungsmesssignal bewirkte Messstrom bzw. die durch das Wechselstrommesssignal bewirkte Messspannung von der Arbeitsspannung und/oder dem Arbeitsstrom Rückfluss durch die Neutralelektrode unterscheiden lassen - beispielsweise durch Auswertung der Frequenz. Somit ist auch in diesem Fall eine ausreichend genaue Bestimmung des wenigstens einen Impedanzbetragswertes und/oder des wenigstens einen Phasenwertes möglich.

Das erfindungsgemäße Verfahren kann beispielsweise unter Verwendung irgendeines Ausführungsbeispiels des Elektrochirurgiesystems durchgeführt werden, wie es vorstehend erläutert wurde. Zunächst wird die Neutralelektrode eines ausgewählten Elektrodentyps am Patienten angebracht, so dass eine elektrisch leitende Verbindung zwischen der Neutralelektrode und dem Patienten besteht. Anschließend wird ein alternierendes Messsignal an die Neutralelektrode angelegt, insbesondere über eine elektrische Verbindung der Neutralelektrode mit den Neutralanschluss eines Versorgungsgeräts. Das Messsignal wird bei wenigstens zwei unterschiedlichen Messfrequenzen angelegt. Basierend auf dem Messsignal wird wenigstens ein Impedanzbetragswert (Scheinwiderstand) einer Neutralelektrodenimpedanz der Neutralelektrode bei zumindest einer der Messfrequenzen ermittelt. Zusätzlich wird bei zumindest einer der Messfrequenzen ein Phasenwert der Neutralelektrodenimpedanz ermittelt. Bei jeder Messfrequenz wird ein Impedanzbetragswert und/oder ein Phasenwert ermittelt.

Anschließend kann basierend auf den wenigstens einen Impedanzbetragswert und dem wenigstens einen Phasenwert der Elektrodentyp der angeschlossenen Neutralelektrode ermittelt werden.

Vorteilhafte Ausgestaltungen der Erfindung ergeben sich aus den abhängigen Ansprüchen, der Zeichnung und der Beschreibung. Nachfolgend werden bevorzugte Ausführungsbeispiele der Erfindung anhand der beigefügten Zeichnungen im Einzelnen erläutert. In der Zeichnung zeigen:

Figur 1 eine schematische, blockschaltbildähnliche Darstellung eines Ausführungsbeispiels eines Elektrochirurgiesystems aufweisend ein Versorgungsgerät, eine Neutralelektrode und ein Instrument,

Figur 2 ein Blockschaltbild eines Ausführungsbeispiels eines Elektrochirurgiesystems,

Figur 3 ein Flussdiagramm eines Ausführungsbeispiels eines erfindungsgemäßen Verfahrens,

Figur 4 eine schematische Prinzipdarstellung eines Ausführungsbeispiels eines ersten Elektrodentyps einer Neutralelektrode,

Figur 5 eine schematische Prinzipdarstellung eines Ausführungsbeispiels eines zweiten Elektrodentyps einer Neutralelektrode,

Figur 6 eine schematische Prinzipdarstellung eines Ausführungsbeispiels eines dritten Elektrodentyps einer Neutralelektrode,

Figur 7 eine schematische, beispielhafte Darstellung von Impedanzbetragswerten der Elektrodentypen gemäß der Figuren 4-6 abhängig von einer Messfrequenz,

Figur 8 schematische, beispielhafte Darstellung von Phasenwerten für die Elektrodentypen gemäß der Figuren 4-6 abhängig von der Messfrequenz,

Figuren 9-11 jeweils einen beispielhaften zeitlichen Verlauf eines Arbeitssignals (Arbeitsspannung und/oder Arbeitsstrom) an einer Arbeitselektrode des Instruments sowie eines Messsignals (Wechselspannungsmesssignal und/oder Wechselstrommesssignal) an einer Neutralelektrode.

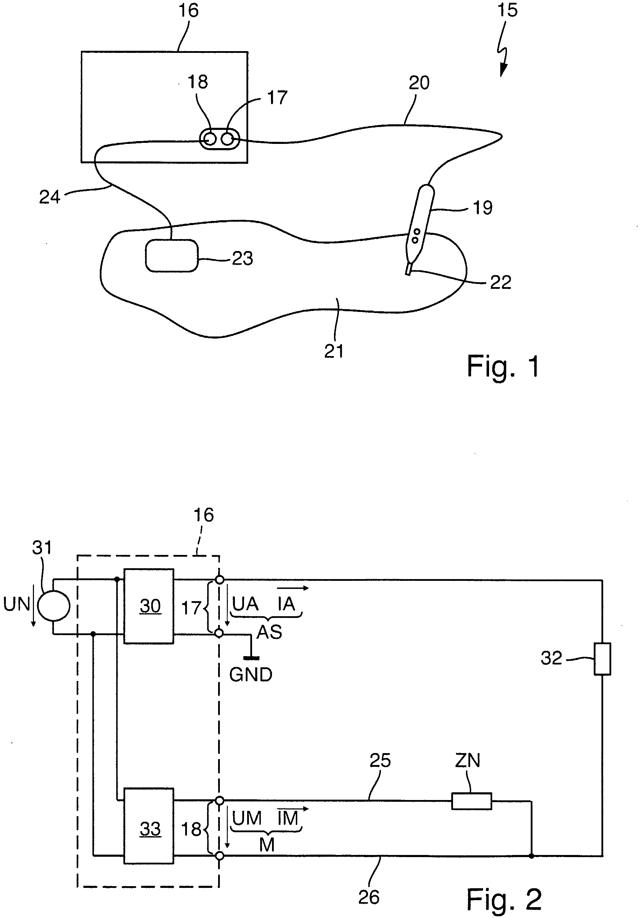

In Figur 1 ist eine Prinzipdarstellung eines Ausführungsbeispiels eines Elektrochirurgiesystems 15 veranschaulicht. Das Elektrochirurgiesystem 15 hat ein Versorgungsgerät 16 mit einem Versorgungsanschluss 17 sowie einem Neutralanschluss 18. Der Versorgungsanschluss 17 dient dazu, ein Instrument 19 elektrisch mit dem Versorgungsgerät 16 zu verbinden. Die elektrische Verbindung kann einpolig oder mehrpolig sein. Die Verbindung kann über ein einadriges oder mehradriges Kabel 20 hergestellt werden.

Das Instrument 19 ist zur elektrochirurgischen Behandlung von biologischem Gewebe 21 eines Patienten eingerichtet. Das Gewebe 21 ist biologisches Gewebe eines menschlichen oder tierischen Körpers. Zur Behandlung des Gewebes 21 hat das Instrument 19 wenigstens eine oder genau eine Arbeitselektrode 22. Zur Behandlung des Gewebes 21 kann eine elektrisch leitende Verbindung zwischen der Arbeitselektrode 22 und dem Gewebe 21 hergestellt werden.

Bei dem in Figur 1 dargestellten Elektrochirurgiesystem 15 ist das Instrument 19 ein monopolares Instrument. Ein Stromkreis zwischen dem Versorgungsgerät 16, der Arbeitselektrode 22 des Instruments 19 und wieder zurück zum Versorgungsgerät 16 wird nicht ausschließlich über das Instrument 19, sondern zusätzlich über eine separate Elektrode hergestellt, die am Patienten elektrisch leitend angebracht wird. Diese zusätzliche Elektrode kann als Neutralelektrode 23 bezeichnet werden. Die Neutralelektrode 23 ist beispielsgemäß über ein mehradriges Kabel 24 mit dem Neutralanschluss 18 des Versorgungsgeräts 16 elektrisch verbunden. Das Kabel 24 hat, z.B. zwei Adern oder Leiter, die beim beschriebenen Ausführungsbeispiel als erster Leiter 25 und zweiter Leiter 26 bezeichnet sind (Figuren 2 und 4-6).

In Figur 2 ist ein Blockschaltbild des Elektrochirurgiesystems 15 gemäß Figur 1 veranschaulicht. Das Versorgungsgerät 16 hat beim Ausführungsbeispiel eine Wechselrichterschaltung 30, die an eine externe Energieversorgung, beispielsweise eine Netzspannungsquelle 31 eines Energieversorgungsnetzes angeschlossen ist. Die Netzspannungsquelle 31 stellt eine Netzspannung für das Versorgungsgerät 16 und beispielsgemäß die Wechselrichterschaltung 30 bereit. Die Wechselrichterschaltung 30 ist dazu eingerichtet, ein Arbeitssignal AS am Versorgungsanschluss 17 bereitzustellen bzw. einzuprägen. Das vom Versorgungsgerät 16 am Versorgungsanschluss 17 bereitgestellte Arbeitssignal AS kann eine eingeprägte Arbeitsspannung UA oder ein eingeprägter Arbeitsstrom IA sein, wobei die Arbeitsspannung UA einen Arbeitsstrom IA verursachen kann bzw. der Arbeitsstrom IA eine Arbeitsspannung UA am Versorgungsanschluss 17 bewirken kann. Das Arbeitssignal AS ist ein Hochfrequenzsignal, d.h. die Arbeitsspannung UA ist eine Hochfrequenzspannung und/oder der Arbeitsstrom IA ist ein Hochfrequenzstrom. Das Arbeitssignal AS wird der Arbeitselektrode 22 während der Behandlung des Gewebes 21 zugeführt. Während der Behandlung fließt der Arbeitsstrom IA über die Arbeitselektrode 22 in das Gewebe 21 zur Neutralelektrode 23 und von dort zurück zum Neutralanschluss 18. Die Gesamtimpedanz dieses Arbeitsstromkreises kann als Arbeitsimpedanz 32 bezeichnet werden.

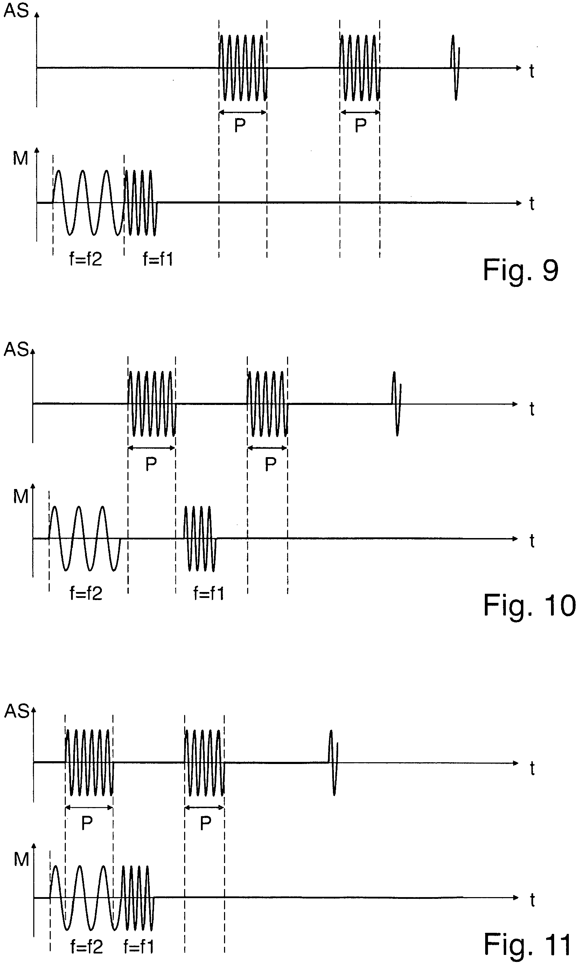

Beim dargestellten Ausführungsbeispiel wird mittels des Instruments 19 über eine Signalleitung des Kabels 20 das Arbeitssignal AS am Versorgungsanschluss 17 angefordert. Während einer oder mehrerer Behandlungsperioden P lieg das Arbeitssignal AS am Versorgungsanschluss 17 dann an der Arbeitselektrode 22 des Instruments an (Figuren 9-11).

Optional kann das Arbeitssignal AS auch dauerhaft am Versorgungsanschluss 17 für das Instrument 19 bereitgestellt werden. Das Instrument 19 kann in diesem Fall eine Schalteinrichtung aufweisen, um basierend auf dem Arbeitssignal AS während der Behandlungsperioden P eine geeignete Spannung und/oder einen geeigneten Strom an der Arbeitselektrode 22 bereitzustellen. Die Schalteinrichtung kann optional eine Wechselrichterschaltung oder eine andere Modifikationsschaltung aufweisen, um das Arbeitssignal AS umzuwandeln oder zu modifizieren, wenn es erforderlich ist.

Auch eine Kombination dieser beschriebenen Ausführungsbeispiele zur Bereitstellung einer geeigneten elektrischen Leistung an der Arbeitselektrode 22 ist möglich.

Die Arbeitsspannung UA entspricht einer Potentialdifferenz zwischen einem Arbeitspotential und einem Bezugspotential, beispielsweise Masse GND.

Das Versorgungsgerät 16 hat beim Ausführungsbeispiel außerdem eine Messeinheit 33. Die Messeinheit 33 ist dazu eingerichtet, ein alternierendes Messsignal M am Neutralanschluss 18 bereitzustellen bzw. einzuprägen. Das Messsignal M kann eine Wechselmessspannung UM oder ein Wechselmessstrom IM sein. Die Wellenform des alternierenden Messsignals M und/oder die Amplitude des Messsignals M können variieren. Das Messsignal M kann beispielsweise sinusförmig bzw. cosinusförmig, dreieckförmig oder rechteckförmig sein. Die Messfrequenz f beträgt vorzugsweise mindestens 100 Hz und maximal 1 MHz.

Abhängig davon, ob die Wechselmessspannung UM oder der Wechselmessstrom IM eingeprägt ist, bewirkt das Messsignal M einen Wechselmessstrom IM durch einen Elektrodenstromkreis oder eine Wechselmessspannung UM am Elektrodenstromkreis. Der Elektrodenstromkreis führt vom ersten Leiter 25 über die Neutralelektrode 23, das Gewebe 21 des Patienten und wieder über die Neutralelektrode 23 und den zweiten Leiter 26 zurück. Die Neutralelektrodenimpedanz ZN im Elektrodenstromkreis ist gekennzeichnet durch einen Impedanzbetragswert $ZN_{abs}$ sowie einen

Phasenwert φ. Für die Neutralelektrodenimpedanz ZN gilt:

$$ZN = ZN_{abs} \cdot e^{j\varphi} \qquad (1)$$

$$ZN = Re(ZN) + j \cdot Im(ZN) \qquad (2)$$

$$ZN_{abs} = \sqrt{Re(ZN)^2 + Im(ZN)^2} \qquad (3)$$

$$\varphi = arctan2(Im(Zn), Re(Zn)) \qquad (4)$$

Re(ZN) ist dabei der Realteil der Neutralelektrodenimpedanz ZN und Im(ZN) ist der Imaginärteil der Neutralelektrodenimpedanz ZN. Der Imaginärteil Im(ZN) und der Realteil Re(Zn) können abhängig von einer Messfrequenz f des alternierenden Messsignals M sein.

Die Messeinheit 33 ist dazu eingerichtet, anhand der Wechselmessspannung UM und des Wechselmessstroms IM für zwei oder mehr Messfrequenzen f jeweils einen Impedanzbetragswert $ZN_{abs}$ und/oder einen Phasenwert φ zu ermitteln. Die Phasenverschiebung zwischen Wechselmessspannung UM und dem Wechselmessstrom IM ergibt sich beispielsgemäß durch kapazitive Effekte im Elektrodenstromkreis, insbesondere durch kapazitive Effekte zwischen der Neutralelektrode 23 und dem Gewebe 21 des Patienten. Diese kapazitiven Effekte werden durch die Neutralelektrodenimpedanz ZN charakterisiert. Die Neutralelektrodenimpedanz ZN beschreibt ebenfalls ohmsche Widerstände und optional auch induktive Effekte im Elektrodenstromkreis. Der Impedanzbetragswert $ZN_{abs}$ kann als Scheinwiderstand bezeichnet werden.

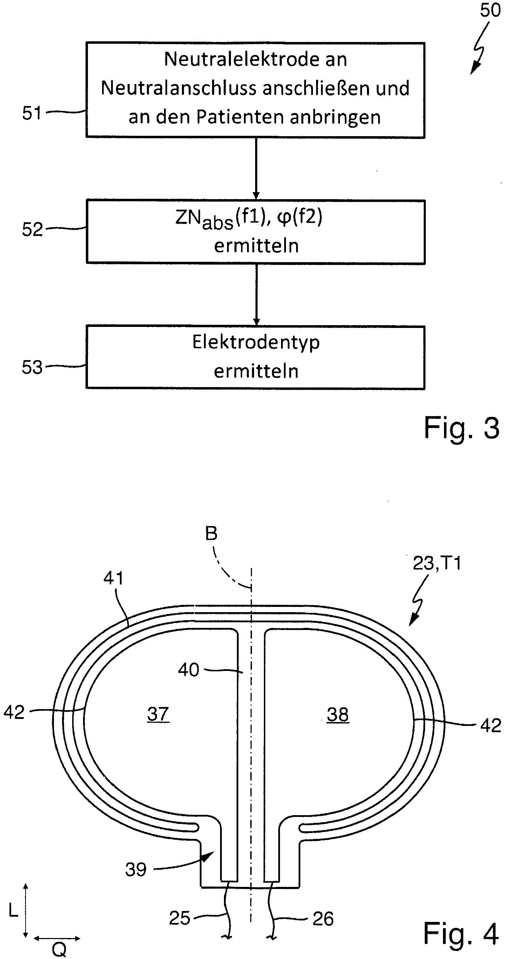

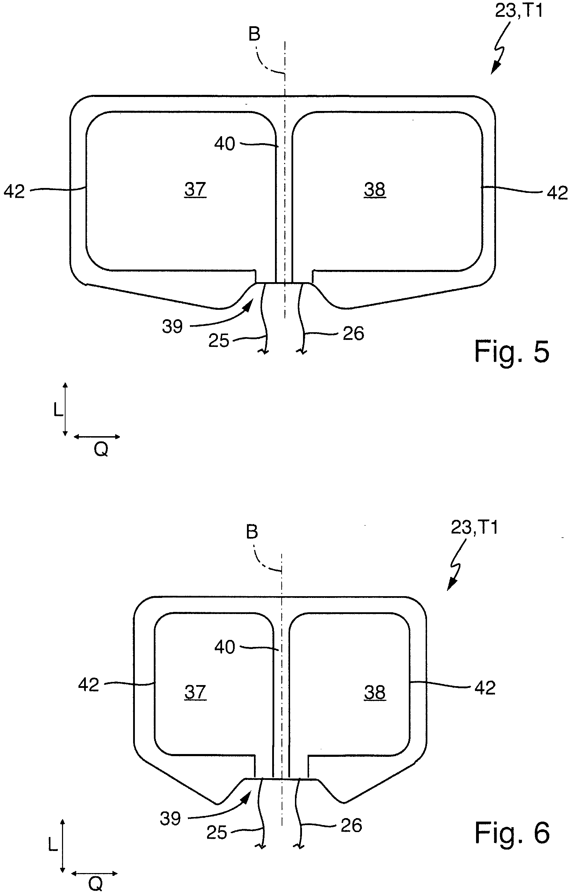

Für das Elektrochirurgiesystem 15 werden unterschiedliche Elektrodentypen von Neutralelektroden 23 bereitgestellt. Lediglich beispielhaft sind hier drei verschiedene Elektrodentypen veranschaulicht, nämlich ein erster Elektrodentyp T1 (Figur 4), ein zweiter Elektrodentyp T2 (Figur 5) sowie ein dritter Elektrodentyp T3 (Figur 6).

Die flächige Neutralelektrode 23 erstreckt sich in einem nicht verformten Ausgangszustand im Wesentlichen parallel zu einer Ebene, die durch eine Längsrichtung L und die Querrichtung Q aufgespannt ist. Die Beschreibung der geometrischen Eigenschaften der Neutralelektrode 23 bezieht sich auf diesen Ausgangszustand.

Beim Ausführungsbeispiel hat jeder der Elektrodentypen T1, T2, T3 wenigstens zwei elektrisch leitfähige Elektrodenbereiche, nämlich einen elektrisch leitfähigen ersten Elektrodenbereich 37 sowie einen elektrisch leitfähigen zweiten Elektrodenbereich 38. In einem Anschlussbereich 39 der Neutralelektrode 23 ist der erste Elektrodenbereich 37 elektrisch mit dem ersten Leiter 25 und der zweite Elektrodenbereich 38 mit dem zweiten Leiter 26 verbunden. Im Anschlussbereich 39 hat sowohl der erste Elektrodenbereich 37, als auch der zweite Elektrodenbereich 38 jeweils eine leiterbahnähnliche Verlängerung bzw. einen leiterbahnähnlichen Fortsatz, an den der zugeordnete Leiter 25 bzw. 26 angeschlossen ist. Diese Verlängerung erstreckt sich bei den hier veranschaulichten Ausführungsbeispielen in Längsrichtung L der Neutralelektrode 23.

Der erste Elektrodenbereich 37 und der zweite Elektrodenbereich 38 jedes Elektrodentyps T1, T2, T3 sind beim Ausführungsbeispiel identisch dimensioniert und haben somit denselben Flächeninhalt und dieselbe Geometrie. Bezüglich einer Bezugsebene B sind der erste Elektrodenbereich 37 und der zweite Elektrodenbereich 38 symmetrisch angeordnet und über einen dazwischen liegenden Steg 40 elektrisch und räumlich voneinander getrennt. Die Bezugsebene B verläuft parallel zur Längsrichtung L und ist rechtwinklig zu einer Querrichtung Q orientiert.

Der erste Elektrodentyp T1 unterscheidet sich von dem zweiten Elektrodentyp T2 und dem Elektrodentyp T3 dadurch, dass zusätzlich zum ersten Elektrodenbereich 37 und dem zweiten Elektrodenbereich 38 ein elektrisch leitfähiger dritter Elektrodenbereich 41 vorhanden ist. Die Neutralelektrode 23 des ersten Elektrodentyps T1 hat beispielsgemäß genau drei Elektrodenbereiche 37, 38, 41, wohingegen die Neutralelektrode 23 des zweiten Elektrodentyps T2 und des dritten Elektrodentyps T3 nur zwei Elektrodenbereiche 37, 38 aufweisen.

Der dritte Elektrodenbereich 41 der Neutralelektrode 23 des ersten Elektrodentyps T1 ist im Unterschied zu den anderen Elektrodenbereichen 37, 38 nicht elektrisch mit dem Versorgungsgerät 16 verbunden oder verbindbar. Sein elektrisches Potential ist daher nicht über das Versorgungsgerät 16 direkt einstellbar.

Sämtliche Elektrodenbereiche 37, 38, 41 aller Elektrodentypen T1, T2, T3 von Neutralelektroden 23 sind innerhalb der Neutralelektrode 23 nicht unmittelbar elektrisch miteinander verbunden, sondern gegenüber den im fehlerfreien Normalbetrieb auftretenden Spannungen und Ströme elektrisch voneinander isoliert. Eine elektrische Verbindung wird in Gebrauchslage über das Gewebe 21 des Patienten mittelbar hergestellt. Die Neutralelektroden 23 sind auf der Seite mit den elektrisch leitfähigen Elektrodenbereichen 37, 38, 41 versehen, die in Gebrauchslage am Gewebe 21 des Patienten angebracht wird. Die entgegengesetzte dem Gewebe 21 abgewandte Rückseite ist vorzugsweise elektrisch isoliert, so dass für Behandlungspersonal ein Berührschutz gegeben ist. Beispielsweise können die Elektrodenbereiche 37, 38, 41 auf ein Trägermaterial der Neutralelektrode 23 aufgebracht werden und auf der Rückseite durch das Trägermaterial oder eine Abdeckschicht abgedeckt und dadurch elektrisch isoliert werden.

Wie es schematisch in Figur 4 veranschaulicht ist, unterscheidet sich der dritte Elektrodenbereich 41 deutlich von der Geometrie der anderen Elektrodenbereiche 37, 38. Er erstreckt sich beispielsgemäß um den ersten Elektrodenbereich 37 und den zweiten Elektrodenbereich 38 herum, wobei er insbesondere ausschließlich im Anschlussbereich 39 geöffnet ist. Die beiden Enden des dritten Elektrodenbereichs 41 liegen sich in Querrichtung Q mit Abstand gegenüber. Zwischen diesen beiden Enden erstreckt sich der dritte Elektrodenbereich 41 unterbrechungslos um den ersten Elektrodenbereich 37 und den zweiten Elektrodenbereich 38 herum. Die Länge des dritten Elektrodenbereichs 41 in seiner Erstreckungsrichtung zwischen seinen beiden Enden ist mindestens um den Faktor 10 oder den Faktor 20 größer als seine Breite rechtwinklig zu dieser Erstreckungsrichtung. Dadurch erhält der dritte Elektrodenbereich 41 eine linienförmige Gestalt.

Die Anzahl der elektrisch leitfähigen Elektrodenbereiche 37, 38, 41 ist daher ein Unterscheidungskriterium zur Unterscheidung unterschiedlicher Elektrodentypen T1, T2, T3. Zusätzlich oder alternativ können sich die Elektrodentypen T1, T2, T3 durch eines oder mehrere der folgenden Unterscheidungsmerkmale voneinander unterscheiden:

- den Flächeninhalt eines, mehrerer oder aller vorhandenen Elektrodenbereiche 37, 38, 41;
- die Kontur oder Geometrie eines, mehrerer oder aller vorhandener Elektrodenbereiche 37, 38, 41;
- das elektrisch leitfähige Material, das für die Elektrodenbereiche 37, 38, 41 verwendet wird;
- die Relativposition der vorhandenen Elektrodenbereiche 37, 38, 41 relativ zueinander.

Bei den veranschaulichten Ausführungsbeispielen ist zu erkennen, dass sich der erste Elektrodentyp T1 vom zweiten Elektrodentyp T2 und dem dritten Elektrodentyp T3 außerdem dadurch unterscheidet, dass der erste Elektrodenbereich 37 und der zweite Elektrodenbereich 38 unterschiedliche Geometrien aufweisen. Beim ersten Elektrodentyp T1 haben der erste Elektrodenbereich 37 und der zweite Elektrodenbereich 38 auf den einander abgewandten Außenseiten jeweils eine bogenförmig und beispielsgemäß kreisbogenförmig verlaufende Außenkante 42. Demgegenüber sind die Außenkanten 42 beim zweiten Elektrodentyp T2 und beim dritten Elektrodentyp T3 zumindest abschnittsweise geradlinig und erstrecken sich beim Ausführungsbeispiel in Längsrichtung L.

Der erste Elektrodenbereich 37 und der zweite Elektrodenbereich 38 haben daher beim ersten Elektrodentyp T1 der Neutralelektrode 23 jeweils im Wesentlichen die Form eines Halbkreises oder eines halben Ovals und beim zweiten Elektrodentyp T2 und beim dritten Elektrodentyp T3 jeweils im Wesentlichen die Form eines Quadrats oder Rechtecks mit abgerundeten Ecken.

Der zweite Elektrodentyp T2 und der dritte Elektrodentyp T3 unterscheiden sich hauptsächlich durch die Dimension der Elektrodenbereiche 37, 38. Beim zweiten Elektrodentyp T2 ist eine Breite der Elektrodenbereiche 37, 38 in Querrichtung Q mindestens so groß wie eine Länge der Elektrodenbereiche 37, 38 in Längsrichtung L. Demgegenüber ist bei dem dritten Elektrodentyp T3 die Länge der Elektrodenbereiche 37, 38 in Längsrichtung L größer als die Breite der Elektrodenbereiche 37, 38 in Querrichtung Q.

Die hier veranschaulichten Elektrodentypen T1, T2, T3 sind lediglich Beispiele für mögliche unterschiedliche Elektrodentypen. Zusätzlich oder alternativ können ein oder mehrere weitere Elektrodentypen vorhanden sein. Beispielsweise können Elektrodentypen verwendet werden, bei denen sich der erste Elektrodenbereich 37 und der zweite Elektrodenbereich 38 in der Geometrie von den hier veranschaulichten Elektrodentypen T1, T2, T3 unterscheidet und beispielsweise andere polygonale Formen mit oder ohne abgerundeten Ecken aufweist.

Erfindungsgemäß wird der an den Neutralanschluss 18 angeschlossene Elektrodentyp T1, T2, T3 automatisch ermittelt. Bei einem erfindungsgemäßen Verfahren 50 (Figur 3) wird in einem ersten Verfahrensschritt 51 die Neutralelektrode 23 des betreffenden Elektrodentyps T1, T2, T3 an den Neutralanschluss 18 des Versorgungsgeräts 16 angeschlossen und am Patienten angebracht, so dass die vorhandenen Elektrodenbereichen 37, 38 und optional 41 elektrisch leitend am Patienten anliegen (insbesondere auf der Haut des Patienten). Die Neutralelektrode 23 kann beispielsweise selbstklebend sein.

In einem zweiten Verfahrensschritt 52 wird anschließend zumindest ein Impedanzbetragswert $ZN_{abs}$ und mindestens ein Phasenwert $\varphi$ ermittelt. Beispielsweise kann es ausreichend sein, einen Impedanzbetragswert $ZN_{abs}$ bei einer ersten Messfrequenz f1 und einen Phasenwert $\varphi$ bei einer zweiten Messfrequenz f2 zu ermitteln, wobei die zweite Messfrequenz f2 kleiner ist als die erste Messfrequenz f1. Die erste Messfrequenz f1 und die zweite Messfrequenz f2 werden vorzugsweise aus einem Frequenzbereich von 100 Hz bis 1,0 MHz ausgewählt. Die erste Messfrequenz f1 kann mindestens 5,0 kHz betragen, beispielsweise 14 bis 15 kHz. Die zweite Messfrequenz f2 beträgt beispielsgemäß maximal 5,0 kHz, vorzugsweise maximal 1,0 kHz. Beim Ausführungsbeispiel wird die zweite Messfrequenz f2 nahe an der unteren Bereichsgrenze des Frequenzbereichs gewählt und kann beispielsweise gleich 100 Hz sein oder im Bereich von 100 Hz bis 200 Hz liegen.

Basierend auf dem wenigstens einen Impedanzbetragswert $ZN_{abs}$ und dem wenigstens einen Phasenwert $\varphi$

kann in einem dritten Verfahrensschritt 53 der Elektrodentyp T1, T2, T3 der angeschlossenen Neutralelektrode 23 ermittelt werden.

Durch die Erkennung des Elektrodentyps T1, T2, T3 der angeschlossenen Neutralelektrode 23 kann das Versorgungsgerät 16 wenigstens einen an den Elektrodentyp T1, T2, T3 angepassten Betriebsmodus vorgeben. Der Betriebsmodus oder einder der möglichen Betriebsmodi kann automatisch eingestellt werden oder manuell vom Bediener ausgewählt werden. Beispielsweise kann abhängig vom ermittelten Elektrodentyp T1, T2, T3 ein Grenzwert für die elektrische Leistung und/oder den durch die Neutralelektrode 23 fließenden Strom verwendet werden. Die Stromdichte im Gewebe 21 kann somit angepasst an die Größe und/oder Geometrie des Elektrodentyps T1, T2, T3 begrenzt werden.

Das Ermitteln des wenigstens einen Impedanzbetragswertes $ZN_{abs}$ und des wenigstens einen Phasenwerts $\varphi$ bei jeweils einer zugeordneten Messfrequenz f1, f2 ist ausreichend. Bei der ersten Messfrequenz f1 und bei der zweiten Messfrequenz f2 wird jeweils ein Impedanzbetragswert $ZN_{abs}$ und/oder ein Phasenwert $\varphi$ ermittelt. In Abwandlung zu dem hier veranschaulichten Ausführungsbeispiel könnten auch mehr als zwei Messfrequenzen f1, f2 zur Ermittlung des wenigstens einen Impedanzbetragswertes $ZN_{abs}$ und des wenigstens einen Phasenwertes $\varphi$ verwendet werden. Die Anzahl der ermittelten Impedanzbetragswerte $ZN_{abs}$ und die Anzahl der ermittelten Phasenwerte $\varphi$ kann gleich groß oder unterschiedlich groß sein. Wenn zwei oder mehr Impedanzbetragswerte $ZN_{abs}$ und/oder zwei oder mehr Phasenwerte $\varphi$ ermittelt werden, werden die zwei oder mehr Impedanzbetragswerte bei unterschiedlichen Messfrequenzen f und/oder die zwei oder mehr Phasenwerte $\varphi$ bei unterschiedlichen Messfrequenzen f1, f2 ermittelt.

Zur Ermittlung des wenigstens einen Impedanzbetragswertes und des wenigstens einen Phasenwertes wird das Messsignal M an den Elektrodenstromkreis und mithin die Neutralelektrode 23 angelegt. Wie erläutert, kann das alternierende Messsignal M eine eingeprägte Wechselmessspannung UM oder ein eingeprägter Wechselmessstrom IM sein. Das Messsignal M wird zumindest bei zwei unterschiedlichen Messfrequenzen f an die Neutralelektrode 23 angelegt, beispielsgemäß bei einer ersten Messfrequenz f1 und einer zweiten Messfrequenz f2 (Figuren 9-11). Dabei kann die Reihenfolge der eingestellten Messfrequenzen f1, f2 beliebig gewählt werden. Bei den in den Figuren 9-11 veranschaulichten schematischen zeitlichen Verläufen des Messsignals M wird lediglich beispielhaft jeweils zunächst die zweite Messfrequenz f2 und anschließend die erste Messfrequenz f1 eingestellt.

Bei den in den Figuren 9 und 10 veranschaulichten Beispielen wird das Messsignal M jeweils außerhalb der Behandlungsperioden P an die Neutralelektrode 23 angelegt. Dadurch wird vermieden, dass sich das Arbeitssignal AS und das Messsignal M gegenseitig beeinträchtigen, da das Arbeitssignal AS ebenfalls einen Stromrückfluss über die Neutralelektrode 23 zum Versorgungsgerät 16 bewirkt.

Alternativ hierzu ist es auch möglich, zumindest zeitweise eine Überlappung des Messsignals M und einer Behandlungsperiode P zuzulassen. In diesem Fall wird die während der Behandlungsperiode P eingestellte Messfrequenz f deutlich unterschiedlich gewählt von der Arbeitsfrequenz des Arbeitssignals AS (Arbeitsspannung UA und/oder Arbeitsstrom IA). Eine derartige zeitliche Überlappung ist beispielhaft in Figur 11 dargestellt.

Das Messsignal M kann im Wesentlichen unterbrechungsfrei zwischen den unterschiedlichen Messfrequenzen f1, f2 umgeschaltet werden (Figuren 9 und 11). Alternativ hierzu kann das Messsignal M auch durch eine Pause unterbrochen werden (Figur 10). Bei diesem Beispiel wird das Messsignal M durch eine oder alternativ auch mehrere Behandlungsperioden P unterbrochen und mit zeitlichem Abstand wird das Messsignal M mit den unterschiedlichen Messfrequenzen f1, f2 an die Neutralelektrode 23 angelegt.

Bei jeder eingestellten Messfrequenz kann durch Auswertung der Wechselmessspannung UM und des Wechselmessstroms IM in der Messeinheit 33 ein Impedanzbetragswert $ZN_{abs}$ und/oder ein Phasenwert $\varphi$ ermittelt werden. Im Anschluss daran kann basierend auf dem wenigstens einen Impedanzbetragswert $ZN_{abs}$ und dem wenigstens einen Phasenwert $\varphi$ ein Vergleich mit vorgegebenen Vergleichswerten für die unterschiedlichen Elektrodentypen T1, T2, T3 durchgeführt und daraus der angeschlossene Elektrodentyp T1, T2, T3 ermittelt bzw. identifiziert werden.

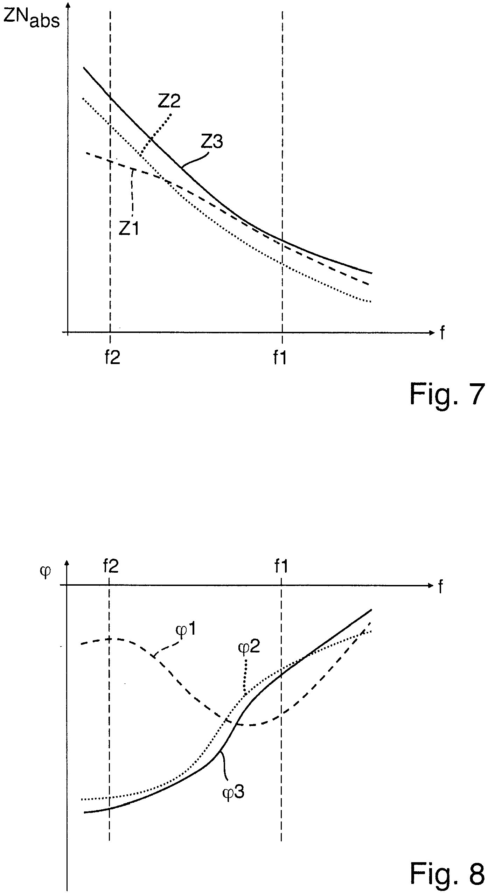

Beispielhaft schematisch sind in den Figuren 7-8 Vergleichswerte in Form von Vergleichskurven dargestellt. Eine erste Impedanzbetragskurve Z1 beschreibt die Impedanzbetragswerte $ZN_{abs}$ abhängig von der Messfrequenz f für den ersten Elektrodentypen T1. Analog hierzu beschreibt die zweite Impedanzbetragskurve Z2 die Impedanzbetragswerte $ZN_{abs}$ für den zweiten Elektrodentypen T2 und die dritte Impedanzbetragskurve Z3 beschreibt die Impedanzbetragswerte $ZN_{abs}$ für den dritten Elektrodentypen T3. Eine erste Phasenkurve $\varphi1$ beschreibt Phasenwerte abhängig von der Messfrequenz f für den ersten Elektrodentypen T1. Analog hierzu beschreibt eine zweite Phasenkurve $\varphi2$ Phasenwerte $\varphi$ für den zweiten Elektrodentypen T2 und eine dritte Phasenkurve $\varphi3$ Phasenwerte $\varphi$ für den dritten Elektrodentypen T3. Die Impedanzbetragskurven Z1, Z2, Z3 sind schematisch und beispielhaft in Figur 7 und die Phasenkurven $\varphi1$, $\varphi2$, $\varphi3$ sind schematisch und beispielhaft in Figur 8 dargestellt. Außerdem sind in den Figuren 7 und 8 die beispielsgemäß verwendeten Messfrequenzen f1, f2 eingetragen.

Wie erläutert kann es ausreichend sein, bei der ersten Messfrequenz f1 den Impedanzbetragswert $ZN_{abs}$ der Neutralelektrodenimpedanz ZN und bei der zweiten Messfrequenz f2 den Phasenwert $\varphi$ der Neutralelektrodenimpedanz ZN zu ermitteln. Anhand der unterschiedlichen Werte kann der unterschiedlichen frequenzabhängigen Werte für den Impedanzbetrag und die Phase dann daraus der angeschlossene Elektrodentyp T1, T2, T3 erkannt werden. Zumindest für die verwendeten Messfrequenzen f1, f2 sind die jeweils zugehörigen Vergleichswerte für den wenigstens einen Impedanzbetragswert $ZN_{abs}$ und den wenigstens einen Phasenwert $\varphi$ vorgegeben, beispielsweise in der Messeinheit 33, so dass durch den Vergleich des ermittelten wenigstens einen Impedanzbetragswert $ZN_{abs}$ und des ermittelten wenigstens einen Phasenwerts $\varphi$ der Elektrodentyp T1, T2, T3 erkannt werden kann.

Es wird nochmals darauf hingewiesen, dass die Impedanzbetragswerte und die Phasenwerte auch jeweils bei mehr als einer Messfrequenz f ermittelt werden können und die Anzahl der ermittelten Impedanzbetragswerte und Phasenwerte variieren kann.

Die Erfindung betrifft ein Elektrochirurgiesystem 15 sowie ein Verfahren 50. Das Elektrochirurgiesystem 15 und das Verfahren 50 sind dazu eingerichtet, einen Elektrodentypen T1, T2, T3 einer verwendeten Neutralelektrode 23 zu ermitteln. Hierzu wird an die Neutralelektrode 23 ein alternierendes Messsignal M bei wenigstens zwei unterschiedlichen Messfrequenzen f1, f2 angelegt. Bei jeder Messfrequenz f1, f2 wird ein Impedanzbetragswert $ZN_{abs}$ oder ein Phasenwert $\varphi$ oder beides für eine Neutralelektrodenimpedanz ZN der Neutralelektrode 23 ermittelt. Es wird zumindest ein Impedanzbetragswert $ZN_{abs}$ und zumindest ein Phasenwert $\varphi$ ermittelt. Basierend auf dem wenigstens einen Impedanzbetragswert $ZN_{abs}$ und dem wenigstens einen Phasenwert $\varphi$ wird - insbesondere durch einen Vergleich mit bekannten Vergleichswerten für den wenigstens einen Impedanzbetragswert $ZN_{abs}$ und für den wenigstens einen Phasenwert $\varphi$ - der Elektrodentyp T1, T2, T3 der angeschlossenen Neutralelektrode 23 ermittelt.

Bezugszeichenliste:

| | |
|---|---|
| 15 | Elektrochirurgiesystem |
| 16 | Versorgungsgerät |
| 17 | Versorgungsanschluss |
| 18 | Neutralanschluss |
| 19 | Instrument |
| 20 | Kabel für das Instruments |
| 21 | Gewebe |
| 22 | Arbeitselektrode |
| 23 | Neutralelektrode |
| 24 | Kabel für die Neutralelektrode |
| 25 | erster Leiter |
| 26 | zweiter Leiter |
| | |
| 30 | Wechselrichterschaltung |
| 31 | Netzspannungsquelle |
| 32 | Arbeitsimpedanz |
| 33 | Messeinheit |
| | |
| 37 | erster Elektrodenbereich |
| 38 | zweiter Elektrodenbereich |
| 39 | Anschlussbereich |
| 40 | Steg |
| 41 | dritter Elektrodenbereich |
| 42 | Außenkante |
| | |
| 50 | Verfahren |
| 51 | erster Verfahrensschritt |
| 52 | zweiter Verfahrensschritt |
| 53 | dritter Verfahrensschritt |
| | |
| $\varphi$ | Phasenwert |
| $\phi1$ | erste Phasenkurve |
| $\varphi2$ | zweite Phasenkurve |
| $\varphi3$ | dritte Phasenkurve |
| | |
| AS | Arbeitssignal |
| B | Bezugsebene |
| f | Messfrequenz |

| f1 | erste Messfrequenz |
|---|---|
| f2 | zweite Messfrequenz |
| GND | Masse |
| IA | Arbeitsstrom |
| IM | Wechselmessstrom |
| L | Längsrichtung |
| M | Messsignal |
| P | Behandlungsperiode |
| Q | Querrichtung |
| t | Zeit |
| T1 | erster Elektrodentyp |
| T2 | zweiter Elektrodentyp |
| T3 | dritter Elektrodentyp |
| UA | Arbeitsspannung |
| UM | Wechselmessspannung |
| Z1 | erste Impedanzbetragskurve |
| Z2 | zweite Impedanzbetragskurve |
| Z3 | dritte Impedanzbetragskurve |
| ZN | Neutralelektrodenimpedanz |
| $ZN_{abs}$ | Impedanzbetragswert |

**Patentansprüche**

1. Elektrochirurgiesystem (15) aufweisend ein Versorgungsgerät (16) mit einem Neutralanschluss (18) und eine an den Neutralanschluss (18) angeschlossene Neutralelektrode (23), die dazu eingerichtet ist, elektrisch leitend mit einem Patienten verbunden zu werden,
wobei das Versorgungsgerät (16) dazu eingerichtet ist, ein alternierendes Messsignal (M) bei wenigstens zwei unterschiedlichen Messfrequenzen (f1, f2) an den Neutralanschluss (18) anzulegen und wenigstens einen Impedanzbetragswert ($ZN_{abs}$) einer Neutralelektrodenimpedanz (ZN) der Neutralelektrode (23) für zumindest eine der Messfrequenzen (f1, f2) zu ermitteln und wenigstens einen Phasenwert ($\varphi$) der Neutralelektrodenimpedanz (ZN) für zumindest eine der Messfrequenzen (f1, f2) zu ermitteln und basierend auf dem wenigstens einen Impedanzbetragswert ($ZN_{abs}$) und dem wenigstens einen Phasenwert ($\varphi$) einen Elektrodentyp (T1, T2, T3) der angeschlossenen Neutralelektrode (23) zu ermitteln.

2. Elektrochirurgiesystem nach Anspruch 1, wobei das Versorgungsgerät (16) dazu eingerichtet ist, für eine erste Messfrequenz (f1) einen Impedanzbetragswert ($ZN_{abs}$) der Neutralelektrodenimpedanz (ZN) zu ermitteln und für eine zweite Messfrequenz (f2) einen Phasenwert ($\varphi$) der Neutralelektrodenimpedanz (ZN) zu ermitteln, wobei die zweite Messfrequenz (f2) kleiner ist als die erste Messfrequenz (f1).

3. Elektrochirurgiesystem nach Anspruch 2, wobei die erste Messfrequenz (f1) im Bereich von 5,0 kHz bis 1,0 MHz liegt.

4. Elektrochirurgiesystem nach Anspruch 2 oder 3, wobei die zweite Messfrequenz (f2) im Bereich von 100 Hz bis 5,0 kHz liegt.

5. Elektrochirurgiesystem nach einem der vorhergehenden Ansprüche, wobei jeder Elektrodentyp (T1, T2, T3) der Neutralelektrode (23) einen elektrisch leitfähigen ersten Elektrodenbereich (37) und einen elektrisch leitfähigen zweiten Elektrodenbereich (38) aufweist, deren elektrischen Potentiale voneinander getrennt sind.

6. Elektrochirurgiesystem nach Anspruch 5, wobei der erste Elektrodenbereich (37) und der zweite Elektrodenbereich (38) symmetrisch zu einer Bezugsebene (B) angeordnet sind.

7. Elektrochirurgiesystem nach Anspruch 5 oder 6, wobei die Neutralelektrode (23) zumindest bei einem der Elektrodentypen (T1) einen elektrisch leitfähigen dritten Elektrodenbereich (41) aufweist, dessen elektrisches Potential getrennt ist von den elektrischen Potentialen des ersten Elektrodenbereichs (37) und des zweiten Elektrodenbereichs (38).

8. Elektrochirurgiesystem nach Anspruch 7, wobei der dritte Elektrodenbereich (41) den ersten Elektrodenbereich (37)

und den zweiten Elektrodenbereich (38) umgibt.

9. Elektrochirurgiesystem nach einem der Ansprüche 5 bis 8, wobei der erste Elektrodenbereich (37) und der zweite Elektrodenbereich (38) zumindest bei einem der Elektrodentypen (T1) der Neutralelektrode (23) auf einander abgewandten Seiten eine bogenförmig gekrümmten Außenkante (42) aufweisen.

10. Elektrochirurgiesystem nach einem der Ansprüche 5 bis 8, wobei der erste Elektrodenbereich (37) und der zweite Elektrodenbereich (37) zumindest bei einem der Elektrodentypen (T2, T3) der Neutralelektrode (23) auf den einander abgewandten Seiten eine abschnittsweise gerade Außenkante (42) aufweisen.

11. Elektrochirurgiesystem nach einem der vorhergehenden Ansprüche, wobei das Versorgungsgerät (16) einem Versorgungsanschluss (17) und ein an den Versorgungsanschluss (17) elektrisch angeschlossenes Instrument (19) mit einer Arbeitselektrode (22) aufweist, wobei das Versorgungsgerät (16) und/oder das Instrument (19) dazu eingerichtet ist, eine Arbeitsspannung (UA) und/oder einen Arbeitsstrom (IA) für die Arbeitselektrode (22) bereitzustellen.

12. Elektrochirurgiesystem nach Anspruch 11, wobei das Versorgungsgerät (16) dazu eingerichtet ist, das Messsignal (M) nur an den Neutralanschluss (18) anzulegen, wenn der Arbeitselektrode (22) keine Arbeitsspannung (UA) und kein Arbeitsstrom (IA) zugeführt wird.

13. Elektrochirurgiesystem nach Anspruch 11, wobei das Versorgungsgerät (16) dazu eingerichtet ist, die Messfrequenz (f1, f2) des Messsignals (M) verschieden zu währen von der Arbeitsfrequenz der Arbeitsspannung (UA) und/oder des Arbeitsstroms (IA), wenn das Messsignal (M) an den Neutralanschluss (18) angelegt wird, während der Arbeitselektrode (22) eine Arbeitsspannung (UA) und/oder ein Arbeitsstrom (IA) zugeführt wird.

14. Elektrochirurgiesystem nach einem der vorherigen Ansprüche, das dazu eingerichtet ist, abhängig von dem erkannten Elektrodentyp (T1, T2, T3) einen dem Elektrodentyp (T1, T2, T3) zugeordneten Betriebsmodus vorzugeben, der manuell oder automatisch einstellbar ist.

15. Verfahren zur Ermittlung eines Elektrodentyps (T1, T2, T3) einer Neutralelektrode (23), das die folgenden Schritte umfasst:

- Anbringen der Neutralelektrode (23) am Patienten, so dass zwischen der Neutralelektrode (23) und dem Patienten eine elektrisch leitende Verbindung besteht,
- Anlegen jeweils eines alternierenden Messsignals (M) bei wenigstens zwei verschiedenen Messfrequenzen (f1, f2) an die Neutralelektrode (23),
- Ermitteln wenigstens eines Impedanzbetragswertes ($ZN_{abs}$) einer Neutralelektrodenimpedanz (ZN) der Neutralelektrode (23) für zumindest eine der Messfrequenzen (f1, f2),
- Ermitteln wenigstens eines Phasenwertes ($\varphi$) der Neutralelektrodenimpedanz (ZN) der Neutralelektrode (23) für zumindest eine der Messfrequenzen (f1, f2),
- Ermitteln eines Elektrodentyps (T1, T2, T3) der angeschlossenen Neutralelektrode (23) basierend auf dem wenigstens einen Impedanzbetragswert ($ZN_{abs}$) und dem wenigstens einen Phasenwert ($\varphi$) .

Fig. 1

Fig. 2

50

| 51 | Neutralelektrode an Neutralanschluss anschließen und an den Patienten anbringen |

| 52 | $ZN_{abs}(f1)$, $\varphi(f2)$ ermitteln |

| 53 | Elektrodentyp ermitteln |

## Fig. 3

## Fig. 4

B                                                    23,T1

42                    37          40          38                    42

39          25          26

L
Q

**Fig. 5**

B                                                    23,T1

42                    37          40          38              42

39          25          26

L
Q

**Fig. 6**

Fig. 7

Fig. 8

Fig. 9

Fig. 10

Fig. 11

Europäisches
Patentamt

European
Patent Office

Office européen
des brevets

# EUROPÄISCHER RECHERCHENBERICHT

Nummer der Anmeldung

EP 22 16 9248

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2020/100825 A1 (HENDERSON JOSHUA [US] ET AL) 2. April 2020 (2020-04-02) | 1-11,14, 15 | INV. A61B18/16 |
| A | * Absätze [0171], [0172], [1784186], [0359]; Abbildungen 2, 3, 25A * * Absätze [0433], [0434] * * Absatz [0507] – Absatz [0525]; Abbildungen 52, 53, 56 * ----- | 12,13 | |
| X | US 2012/232548 A1 (BEHNKE II ROBERT J [US] ET AL) 13. September 2012 (2012-09-13) | 1-11,14, 15 | |
| A | * das ganze Dokument * ----- | 12,13 | |
| X | US 2010/331835 A1 (SHILEV NICKOLAY D [BG]; BOVIE MEDICAL CORP [US]) 30. Dezember 2010 (2010-12-30) | 1-10,15 | |
| A | * das ganze Dokument * ----- | 12,13 | |
| A | US 2011/202055 A1 (SELIG PETER [DE]) 18. August 2011 (2011-08-18) * Abbildungen 1-3 * * Absätze [0016], [0017] * * Absatz [0024] – Absatz [0026] * ----- | 1-15 | RECHERCHIERTE SACHGEBIETE (IPC) A61B |
| A | US 2009/234352 A1 (BEHNKE ROBERT J [US] ET AL) 17. September 2009 (2009-09-17) * Absatz [0009] – Absatz [0012]; Abbildungen 1-4 * ----- | 1-15 | |

Der vorliegende Recherchenbericht wurde für alle Patentansprüche erstellt

| Recherchenort | Abschlußdatum der Recherche | Prüfer |
|---|---|---|
| München | 7. Oktober 2022 | Lorenz, Larissa |

**ANHANG ZUM EUROPÄISCHEN RECHERCHENBERICHT
ÜBER DIE EUROPÄISCHE PATENTANMELDUNG NR.**

EP 22 16 9248

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07−10−2022

| Im Recherchenbericht angeführtes Patentdokument | Datum der Veröffentlichung | Mitglied(er) der Patentfamilie | Datum der Veröffentlichung |
|---|---|---|---|
| US 2020100825 A1 | 02−04−2020 | BR 112021003060 A2 | 11−05−2021 |
| | | BR 112021003347 A2 | 11−05−2021 |
| | | BR 112021003372 A2 | 11−05−2021 |
| | | BR 112021003376 A2 | 18−05−2021 |
| | | BR 112021003428 A2 | 18−05−2021 |
| | | BR 112021003595 A2 | 18−05−2021 |
| | | BR 112021003621 A2 | 18−05−2021 |
| | | BR 112021003781 A2 | 18−05−2021 |
| | | BR 112021003824 A2 | 25−05−2021 |
| | | BR 112021003893 A2 | 18−05−2021 |
| | | BR 112021003932 A2 | 18−05−2021 |
| | | BR 112021003938 A2 | 18−05−2021 |
| | | BR 112021004237 A2 | 18−05−2021 |
| | | CN 112638298 A | 09−04−2021 |
| | | CN 112638299 A | 09−04−2021 |
| | | CN 112654310 A | 13−04−2021 |
| | | CN 112654315 A | 13−04−2021 |
| | | CN 112654316 A | 13−04−2021 |
| | | CN 112654317 A | 13−04−2021 |
| | | CN 112654318 A | 13−04−2021 |
| | | CN 112654319 A | 13−04−2021 |
| | | CN 112654322 A | 13−04−2021 |
| | | CN 112654323 A | 13−04−2021 |
| | | CN 112672705 A | 16−04−2021 |
| | | CN 112672706 A | 16−04−2021 |
| | | CN 112689481 A | 20−04−2021 |
| | | EP 3846705 A1 | 14−07−2021 |
| | | EP 3846710 A1 | 14−07−2021 |
| | | EP 3846711 A1 | 14−07−2021 |
| | | EP 3846712 A1 | 14−07−2021 |
| | | EP 3846714 A1 | 14−07−2021 |
| | | EP 3846715 A1 | 14−07−2021 |
| | | EP 3846719 A1 | 14−07−2021 |
| | | EP 3846720 A1 | 14−07−2021 |
| | | EP 3846721 A1 | 14−07−2021 |
| | | EP 3846722 A1 | 14−07−2021 |
| | | EP 3846723 A1 | 14−07−2021 |
| | | EP 3846728 A1 | 14−07−2021 |
| | | EP 3846730 A1 | 14−07−2021 |
| | | JP 2021536311 A | 27−12−2021 |
| | | JP 2021536312 A | 27−12−2021 |
| | | JP 2021536314 A | 27−12−2021 |
| | | JP 2021536315 A | 27−12−2021 |
| | | JP 2021536316 A | 27−12−2021 |
| | | JP 2021536317 A | 27−12−2021 |
| | | JP 2021536318 A | 27−12−2021 |

EPO FORM P0461

Für nähere Einzelheiten zu diesem Anhang : siehe Amtsblatt des Europäischen Patentamts, Nr.12/82

Seite 1 von 3

**ANHANG ZUM EUROPÄISCHEN RECHERCHENBERICHT
ÜBER DIE EUROPÄISCHE PATENTANMELDUNG NR.**

`EP 22 16 9248`

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

`07-10-2022`

| Im Recherchenbericht angeführtes Patentdokument | Datum der Veröffentlichung | Mitglied(er) der Patentfamilie | | Datum der Veröffentlichung |
|---|---|---|---|---|
| | | JP | 2021536321 A | 27-12-2021 |
| | | JP | 2021536322 A | 27-12-2021 |
| | | JP | 2021536323 A | 27-12-2021 |
| | | JP | 2022500110 A | 04-01-2022 |
| | | JP | 2022500111 A | 04-01-2022 |
| | | JP | 2022517460 A | 09-03-2022 |
| | | US | 2020078070 A1 | 12-03-2020 |
| | | US | 2020078076 A1 | 12-03-2020 |
| | | US | 2020078077 A1 | 12-03-2020 |
| | | US | 2020078078 A1 | 12-03-2020 |
| | | US | 2020078079 A1 | 12-03-2020 |
| | | US | 2020078080 A1 | 12-03-2020 |
| | | US | 2020078081 A1 | 12-03-2020 |
| | | US | 2020078082 A1 | 12-03-2020 |
| | | US | 2020078089 A1 | 12-03-2020 |
| | | US | 2020078106 A1 | 12-03-2020 |
| | | US | 2020078110 A1 | 12-03-2020 |
| | | US | 2020078111 A1 | 12-03-2020 |
| | | US | 2020078112 A1 | 12-03-2020 |
| | | US | 2020078114 A1 | 12-03-2020 |
| | | US | 2020078115 A1 | 12-03-2020 |
| | | US | 2020078116 A1 | 12-03-2020 |
| | | US | 2020078117 A1 | 12-03-2020 |
| | | US | 2020078118 A1 | 12-03-2020 |
| | | US | 2020078119 A1 | 12-03-2020 |
| | | US | 2020100825 A1 | 02-04-2020 |
| | | US | 2020100830 A1 | 02-04-2020 |
| | | WO | 2020051439 A1 | 12-03-2020 |
| | | WO | 2020051442 A1 | 12-03-2020 |
| | | WO | 2020051443 A1 | 12-03-2020 |
| | | WO | 2020051444 A1 | 12-03-2020 |
| | | WO | 2020051446 A1 | 12-03-2020 |
| | | WO | 2020051448 A1 | 12-03-2020 |
| | | WO | 2020051449 A1 | 12-03-2020 |
| | | WO | 2020051457 A1 | 12-03-2020 |
| | | WO | 2020051462 A1 | 12-03-2020 |
| | | WO | 2020051463 A1 | 12-03-2020 |
| | | WO | 2020051466 A1 | 12-03-2020 |
| | | WO | 2020051471 A1 | 12-03-2020 |
| | | WO | 2020051472 A1 | 12-03-2020 |
| | | WO | 2020051474 A1 | 12-03-2020 |
| | | WO | 2020051475 A1 | 12-03-2020 |
| | | WO | 2020051478 A1 | 12-03-2020 |
| US 2012232548 A1 | 13-09-2012 | AU | 2009200404 A1 | 20-08-2009 |
| | | CA | 2652193 A1 | 04-08-2009 |

Für nähere Einzelheiten zu diesem Anhang : siehe Amtsblatt des Europäischen Patentamts, Nr.12/82

EPO FORM P0461

`Seite 2 von 3`

**ANHANG ZUM EUROPÄISCHEN RECHERCHENBERICHT
ÜBER DIE EUROPÄISCHE PATENTANMELDUNG NR.**

EP 22 16 9248

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-10-2022

| Im Recherchenbericht angeführtes Patentdokument | Datum der Veröffentlichung | Mitglied(er) der Patentfamilie | Datum der Veröffentlichung |
|---|---|---|---|
| | | EP 2085044 A1 | 05-08-2009 |
| | | JP 5455388 B2 | 26-03-2014 |
| | | JP 2009183708 A | 20-08-2009 |
| | | US 2009198230 A1 | 06-08-2009 |
| | | US 2012232548 A1 | 13-09-2012 |
| US 2010331835 A1 | 30-12-2010 | KEINE | |
| US 2011202055 A1 | 18-08-2011 | CN 102202592 A | 28-09-2011 |
| | | DE 102009013917 A1 | 12-05-2010 |
| | | EP 2367493 A1 | 28-09-2011 |
| | | JP 5476390 B2 | 23-04-2014 |
| | | JP 2012506744 A | 22-03-2012 |
| | | PL 2367493 T3 | 27-02-2015 |
| | | US 2011202055 A1 | 18-08-2011 |
| | | WO 2010049145 A1 | 06-05-2010 |
| US 2009234352 A1 | 17-09-2009 | AU 2009201070 A1 | 01-10-2009 |
| | | CA 2658407 A1 | 17-09-2009 |
| | | EP 2103269 A1 | 23-09-2009 |
| | | JP 2009233325 A | 15-10-2009 |
| | | US 2009234352 A1 | 17-09-2009 |

EPO FORM P0461

Für nähere Einzelheiten zu diesem Anhang : siehe Amtsblatt des Europäischen Patentamts, Nr.12/82

Seite 3 von 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2537479 A1
- EP 1289415 A1
- EP 0813387 A1
- WO 03060462 A2
- EP 3496638 A1
- DE 102019209333 A1